Europäisches Patentamt

European Patent Office

Office européen des brevets

(19)

(11) Publication number: **0 084 975**
**B1**

(12) **EUROPEAN PATENT SPECIFICATION**

(45) Date of publication of patent specification: **30.12.86**

(21) Application number: **83300361.9**

(22) Date of filing: **25.01.83**

(51) Int. Cl.$^4$: **C 12 P 19/32,** C 12 P 1/00,
C 12 M 1/40 // C12P21/02,
C12P13/20

(54) Process for producing physiologically active substance by multienzyme process and apparatus for the same.

(30) Priority: 26.01.82 JP 10338/82
27.05.82 JP 90424/82
09.12.82 JP 216207/82

(43) Date of publication of application:
03.08.83 Bulletin 83/31

(45) Publication of the grant of the patent:
30.12.86 Bulletin 86/52

(84) Designated Contracting States:
CH DE FR GB IT LI SE

(56) References cited:
EP-A-0 050 007
US-A-4 164 444

CHEMICAL ABSTRACTS, vol. 87, no. 3, 18th
July 1977, page 241, no. 18202y, Columbus,
Ohio, US

EUROPEAN JOURNAL OF APPLIED
MICROBIOLOGY AND BIOTECHNOLOGY, vol.
10, 1980, pages 11-21, Springer-Verlag, DE K.
MURATA et al.: "Glutathione production
coupled with an ATP regeneration system"

(73) Proprietor: Imahori, Kazutomo
No.2-25-23, Kakinokisaka
Meguro-ku Tokyo (JP)

(73) Proprietor: RIKAGAKU KENKYUSHO
2-1 Hirosawa
Wako-shi Saitama-ken (JP)

(73) Proprietor: UNITIKA LTD.
No. 50, Higashihonmachi 1-chome
Amagasaki-shi Hyogo (JP)

(72) Inventor: Kazutomo, Imahori
2-25-23 Kakinokisaka
Meguro-ku Tokyo (JP)
Inventor: Isao, Tomioka
16-1 Uryu Terado-cho
Muko-shi Kyoto (JP)
Inventor: Tatsuo, Iwasaki
407 Higashiuratsuji-cho Schimodachiurisagaru
Nishitoutin Kamigyo-ku Kyoto (JP)
Inventor: Masaru, Kashima
29-19 Kitakuzuha-cho
Hirakata-shi Osaka (JP)
Inventor: Hiroshi, Nakajima
80-4, Enba Makishima-cho
Uji-shi Kyoto (JP)
Inventor: Toshihiko, Tsukamoto
2-220 Gokashohirookatani
Uji-shi Kyoto (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 084 975**

(56) References cited:
CHEMICAL ABSTRACTS, vol. 83, no. 5, 4th
August 1975, page 201, no. 39469y, Columbus,
Ohio, US G.M. WHITESIDES et al.: "Enzymic
regeneration of ATP from AMP and ADP. II.
Enzyme immobilization and reactor
development"

JOURNAL OF THE AMERICAN CHEMICAL
SOCIETY, vol. 100, no. 1, 4th January 1978,
pages 304-306, Gaston, PA., US R.L. BAUGHN
et al.: "Large-scale enzyme-catalyzed synthesis
of ATP from adenosine and acetyl phosphate.
Regeneration of ATP from AMP"

AIChE JOURNAL, vol. 22, no. 6, November
1976, pages 1079-1090 R.S. LANGER et al.:
"Enzymatic regeneration of ATP"

(72) Inventor: **Hitoshi, Kondo**
**38, Ujiwakamori**
**Uji-shi Kyoto (JP)**

(74) Representative: **Pearce, Anthony Richmond**
**et al**
**MARKS & CLERK Alpha Tower Suffolk Street**
**Queensway Birmingham B1 1TT (GB)**

# Description

## Field of the invention

The present invention relates to a process for converting adenosine - 5' - monophosphate (hereinafter referred to as AMP) into adenosine -, 5' - triphosphate (hereinafter referred to as ATP), an apparatus for the same and a process for producing a physiologically active substance by an enzymic reaction using ATP as an auxiliary factor.

## Background of the invention

In recent years, chemical reactions in living bodies have been closely examined in order to attempt to reproduce the chemical reactions in living bodies in a reactor. In living bodies, many biosynthesis reactions are naturally performed in the presence of enzymes as a catalyst in order to support life. Accordingly, living bodies easily produce compounds which are difficult to synthesize by chemical reactions. Knowledge of such reactions is becoming important for satisfying social requirements such as conserving energy and eliminating public nuisances. Reproducing such reactions will likely become an essential technique in chemical industries. The practical use of such reactions has already been found useful in the technical fields of hydrolysis and isomerization.

When carrying out a synthesis reaction (which is a particularly important reaction in biosynthesis reactions), ATP is required as an energy source or an auxiliary factor. In such reactions, ATP is consumed by decomposing into adenosine - 5' - diphosphate (hereinafter referred to as. ADP) or AMP and thereby serves as an energy source or an auxiliary factor. Accordingly, in order to industrially reproduce the synthesis reaction, it is necessary to supply ATP at a moderate price. However, ATP is a very expensive substance. Accordingly, it is important to convert ADP and, particularly, AMP which are present after the consumption of ATP, into ATP.

Many studies concerning reproduction and conversion into ATP have been conducted. For example, since production of ATP is carried out by a glycolysis reaction in the living bodies, an attempt utilizing such a reaction is known in T. Tochikura, M. Kuwahara, S. Yagi, H. Okamoto, Y. Tominaga, T. Kano and K. Ogata, *J. Ferment. Tech., 45,* 511 (1967); H. Samejima, K. Kimura, Y. Ado, Y. Suzuki and T. Tadokoro, *Enzyme Eng., 4,* 237 (1978) and M. Asada, K. Yanamoto, K. Nakanishi, R. Matsuno and T. Kamikubo, *Eur. J. Appl, Microbial. Biotechnol., 12,* 198 (1981). The concept of the reaction is that reproduction and supply of consumed ATP are carried out using microorganisms, wherein AMP or adenosine is used as a raw material for ATP. However, since the AMP or adenosine employed is not one which has resulted from the consumption of ATP, it is specifically added as a new source of ATP. Under these circumstances, the conversion efficiency of AMP or adenosine into ATP is very inferior and side reactions occur. Specifically, when glycolysis using microorganisms is effected, negative results are obtained concerning effective reproduction of ATP from AMP which has been produced as a result of consumption of ATP.

The use of an ATP conversion enzyme which is not a thermostable enzyme has been attempted. Langer et al. have reported process in converting AMP into ATP by means of adenylate kinase in rabbit muscles and acetate kinase in *Escherichia coli* in R. S. Langer, B. K. Hamilton, C. R. Gardner, M. C. Archer and C. K. Colton, *AlchE J., 22,* 1079 (1976); R. S. Langer, C. R. Gardner, B. K. Hamilton and C. K. Colton, *AlchE J., 23,* 1 (1977), and U.S. Patent 4,164,444. Further, the conversion of adenosine into ATP using adenosine kinase has also been reported in R. L. Baughn, O. Adalsteinsson and G. M. Whitesides, *J. Am. Chem. Soc., 100,* 304 (1978). Furthermore, Whitesides et al. have reported that, when the above-described adenylate kinase and acetate kinase are immobilized on Sepharose (Registered Trade Mark) with cyanogen bromide to continuously convert AMP into ATP, the residual activity is low in the absence of a stabilizer, and stability with the passage of time is very inferior [G. M. Whitesides, A. Chmurny, P. Garrett and C. K. Colton, *Enzyme Eng., 2,* 217 (1974)]. Moreover, even if an immobilized enzyme is used and a stabilizer is added, the reaction requires a long period of time and conversion efficiency is not very high, and it cannot be utilized in an industrial environment for extended periods of time.

However, little is known with respect to the production of useful substances by the above-described synthesis reaction involving reproducing ATP. There is a process which comprises reproducing ATP which has been consumed during the synthesis of glutathione by reaction of glutamic acid, cystein and glycine with γ-glutamyl cystein synthesis enzyme and glutathione synthesis enzyme, from ADP which has been produced as a result of the consumption of ATP, said reproduction of ATP being effected by acetate kinase originating from *Escherichia coli,* and the reproduced ATP being used again (as described in K. Murata, K. Tani, J. Kato and I. Chibata, *Eur. J. Microbial Biotechnol., 10,* 11 (1980)). However, this process does not provide any information with regard to the conversion of AMP back to ATP.

A bioreactor for synthesizing a useful substance by continuously consuming ATP to form AMP has been considered, and it has been highly desired to complete such a system as described in G. M. Whitesides, A. Chmurny, P. Garrett, L. Lamotte and C. K. Colton, *Enzyme Eng., 2,* 217 (1974).

## Summary of the invention

An object of the present invention is to provide a process for converting AMP (which is a product obtained by decomposing ATP to the lowest energy level) into ATP in a high yield and an apparatus for the same. Another object of the present invention is to provide a process for producting a physiologically active substance by a multienzyme process which comprises using AMP which has been obtained by decomposition to the lowest energy level, as a raw material.

As result of earnest studies to attain the above-described objects, the present inventors have found that AMP can be converted into ATP in a high yield in a short period of time, when conversion enzymes produced from microorganisms having an optimum growth temperature of 50°C to 85°C are used. As a result of subsequent studies, we have found, on the basis of the above-described knowledge, that a physiologically active substance can be synthesised from AMP which is a product obtained by decomposition of ATP to the lowest energy level, as a raw material, by combining (a) a reaction system of converting AMP into ATP with (b) a reaction system of synthesising a physiologically active substance from ATP.

The present invention relates to a process for converting AMP into ATP which comprises using, as conversion enzymes, an enzyme which converts AMP into ADP, the enzyme having been produced from microorganisms having an optimum growth temperature of 50°C to 85°C, and an enzyme which converts ADP into ATP, the enzyme having been produced from micr-organisms having an optimum growth temperature of 50°C to 85°C.

The present invention also relates to an apparatus for converting AMP into ATP which comprises:

(a) an enzyme reactor having outlet conduit means and inlet conduit means, wherein said enzyme reactor contains:

(1) an enzyme which converts AMP into ADP, wherein said enzyme has been produced from microorganisms having an optimum growth temperature of 50°C to 85°C, and also contains

(2) an enzyme which converts ADP into ATP, wherein said enzyme has been produced from microorganisms having an optimum growth temperature of 50°C to 85°C;

(b) a source of AMP, wherein said source is connected to said enzyme reactor by said inlet conduit means;

(c) a source of phosphoric acid donor for converting AMP into ADP, wherein said source is connected to said enzyme reactor by said inlet conduit means;

(d) a source of phosphoric acid donor for converting ADP into ATP, wherein said source is connected to said enzyme reactor by said inlet conduit means;

(e) at least one variable fluid-delivery apparatus connected between each of said sources (b) (c) and (d), and said enzyme reactor, wherein said at least one variable fluid-delivery apparatus controls the flow of AMP from said source of AMP (b), the flow of phosphoric acid donor from said source of phosphoric acid donator (c) and the flow of phosphoric acid donor from said source of phosphoric acid donor (d), into said enzyme reactor;

(f) a recovery apparatus connected to said enzyme reactor outlet conduit means through;

(g) an automatic sampling apparatus;

(h) an analyzing apparatus which analyzes the concentrations of ATP, ADP and AMP in the reaction solution flowing from said enzyme reactor, wherein said analyzing apparatus provides signals indicative of said concentrations to;

(i) an arithmetical control apparatus which on the basis of signals received from said analyzing apparatus (h) and signals of previously precribed values stored therein, provides signals to said at least one variable fluid sending apparatus (e) to control the amount of flow from said sources (b), (c) and (d) to said enzyme reactor so as to maintain the ATP conversion in said enzyme reactor or the ATP concentration in said enzyme reactor outlet conduit means at previously prescribed values.

Furthermore, the invention relates to a process for producing a physiologically active substance by a multienzyme process which comprises (a) forming ATP from AMP using a combination of an enzyme which converts AMP into ADP and which has been produced from microorganisms having an optimum growth temperature of 50°C to 85°C, and an enzyme which converts ADP into ATP and which has been produced from microorganisms having an optimum growth temperature of 50°C to 85°C, (b) synthesizing a physiologically active substance with the resulting ATP, converting AMP resulting from the reaction in the step (b) into ATP by the reaction in the step (a), and repeatedly utilizing the converted ATP for synthesis of the physiologically active substance in step (b).

According to the present invention, it becomes possible to carry out conversion of AMP into ATP efficiently over a long period of time in a stable manner. Further, it is possible to continuously and economically carry out an enzymic reaction using ATP as an auxiliary factor with very good efficiency, whereby it becomes possible to realize operation of the so-called bioreactor wherein synthesis reactions in the living body are carried out as industrial chemical reactions outside the living body.

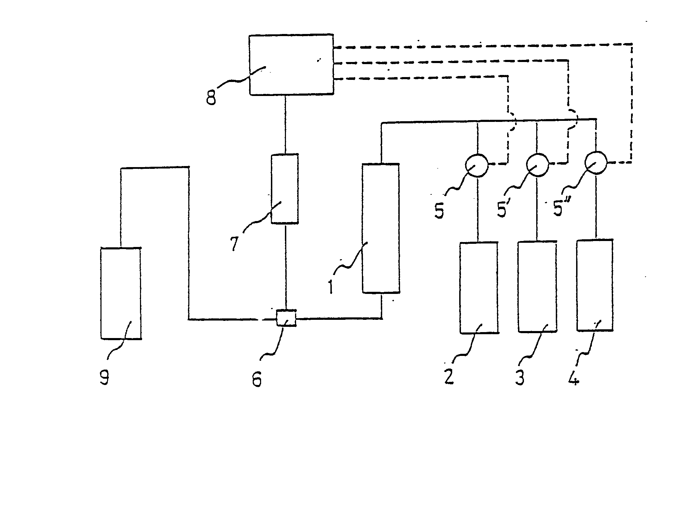

Brief description of the drawing

The drawing is a schematic illustration of an embodiment of an apparatus for converting AMP into ATP according to the present invention.

Detailed description of the invention

The present invention comprises conversion of AMP into ADP and conversion of the resulting ADP into ATP. For example, adenylate kinase is used as an enzyme for converting AMP into ADP, and ATP is used as a phosphoric acid donor in this case. Examples of enzymes for converting ADP into ATP include acetate kinase, carbamate kinase, creatine kinase, 3 - phosphoglycerate kinase, pyruvate kinase and polyphosphate kinase. Acetate kinase is preferably used considering the price of the phosphoric acid donor, the activity of conversion into ATP and the availability of enzymes, etc. In this case, acetylphosphate is used as a phosphoric acid donor. As described above, though ATP and acetyl phosphate are used as phosphoric acid donors when using adenylate kinase and acetate kinase, it is sufficient to supply only acetyl phosphate as the phosphoric acid donor, because ATP as the final converion product can be used as a phosphoric acid donor. From the application of the above-described advantages which are obtained when using acetate kinase alone and when using a combination of acetate kinase and adenylate kinase, it becomes possible to plan a system in which the ATP is effectively reproduced from the AMP.

As described above, it becomes possible to convert AMP into ATP using two kinds of conversion enzyme. However, these enzymes are those produced from microorganisms having an optimum growth temperature of 50°C to 85°C. Examples of such microorganisms include microorganisms of the genus *Bacillus*, such as *Bacillus stearothermophillus*, *Bacillus brevis*, *Bacillus coagulans*, *Bacillus thermoproteolyticus* or *Bacillus acidocaldarius*, etc., microorganisms of the genus *Clostridium*, microorganisms of the genus *Thermoactinomyces*, microorganisms of the genus *Achromobacter*, microorganisms of the genus *Streptomyces*, microorganisms of the genus *Micropolyspora*, microorganisms of the genus *Thermus* such as *Thermus aquaticus*, *Thermus thermophilus* or *Thermus flavus*, etc., microorganisms of the genus *Thermomicrobium*, etc. Further, there are microorganisms growing at a normal temperature into which genes of the above-described microorganisms are introduced. Among these microorganisms, *Bacillus stearothermophillus* is particularly suitable for producing both adenylate kinase and acetate kinase. Both enzymes obtained from this microorganism can be easily purified and have a high specific activity. In the present invention, it is preferred to use the above-described enzymes in an immobilized state. For this purpose, the enzymes are bonded to, included in or absorbed in suitable carriers. Examples of such carriers include polysaccharide derivatives such as cellulose, dextran or agarose, etc., vinyl polymer derivatives such as polystyrene, ethylenemaleic acid copolymer or cross-linked polyacrylamide, etc., polyamino acids and polyamide derivatives such as L-alanine - L - glutamic acid copolymer or polyaspartic acid, etc., and inorganic derivatives such as glass, alumina or hydroxyapatite, etc., preferably polysaccharide derivatives, inorganic derivatives such as glass and vinyl polymer derivatives such as polystyrene, which are used by packing a reactor such as a column therewith. The preferred amount of the above carriers used per the enzyme is 1 µg/enzyme unit to 100 g/enzyme unit (indicated by enzyme activity unit), more preferably 10 µg/enzyme unit to 10 g/enzyme unit.

As an apparatus used for converting AMP into ATP in the present invention, there is, for example, an apparatus which is schematically illustrated in the accompanying drawing.

This apparatus is composed of an enzyme reactor 1 including an enzyme for converting AMP into ADP and an enzyme for converting ADP into ATP, a source 2 of AMP, a source 3 of phosphoric acid donor for converting AMP into ADP, a source 4 of phosphoric acid donor for converting ADP into ATP, variable fluid-delivery apparatus 5, 5' and 5'' which are arranged and connected so that AMP and the above-described phosphoric acid donors are fed from each source 2, 3 and 4 to one end of the enzyme reactor 1 to cause enzymic reacting and a reacting solution to flow continuously from the other end, an automatic sampling apparatus 6 and an analyzing apparatus 7 for the reacting solution which are arranged and connected so as to automatically analyze ATP, ADP and AMP in the reacting solution from the enzyme reactor 1, an arithmetical control apparatus 8 which is arranged and connected so as to control the amount of flow in at least one of the above-described variable fluid-delivery apparatus 5, 5' and 5'' by which arithmetical operation is carried out on the basis of data signals received from the above-described analyzing apparatus 7 and signals of the prescribed values previously fed so as to keep the ATP conversion in the above-described enzyme reactor 1 or the ATP concentration in the outlet of said reactor at a previously prescribed value, and a recovery apparatus 9 for recovering the reacting solution flowing from the enzyme reactor 1.

In order to convert AMP into ATP using the above-described apparatus according to the present invention, it is preferred to carry out conversion of AMP→ADP→ATP in a packed bed reactor by feeding 0.1 µM to 4 M, preferably 1 µM to 2 M and, more preferably 10 µM to 500 mM of AMP (source of AMP supply 2), 0.1 µM to 500 mM, preferably 1 µM to 400 mM and, more preferably 10 µM to 300 mM of acetyl phosphate (source 4 of phosphoric acid donor supply for converting ADP into ATP) and ATP (source 3 of phosphoric acid donor supply for converting AMP into ADP) to an end of the reactor 1. In this case, it is particularly preferred to use ATP in an amount satisfying the formula (a).

$$0.15 \times \frac{5+r^2}{r^2} \times AMP \geqq ATP \geqq 0.04 \times \frac{5+r^2}{r^2} \times AMP$$

$$(a)$$

(wherein AMP represents the concentration of AMP (mM), ATP represents the concentration of ATP (mM), and r represents a ratio of immobilized enzyme activity of the enzyme which converts ADP into ATP to immobilized enzyme activity of the enzyme which converts AMP into ADP, which is a positive number of 1 or more).

The reacting solution eluted from the reactor in this case can be analyzed by the analyzing apparatus 7 to determine the concentrations of AMP, ADP and ATP and the conversion to ATP. In this case, the flow rate varies according to the size of the reactor. For example, a suitable flow rate can be selected from the linear velocity range of $1\times10^{-4}$ cm/hr to $1\times10^{6}$ m/hr. When using a 5 liter reactor having an inside diameter of 10 cm and a length of 63.5 cm, it is preferred to select a suitable flow rate from a linear velocity range of $6\times10^{-2}$ cm/hr to $1\times10^{5}$ cm/hr. The apparatus for supplying AMP, ATP and acetyl phosphate to the reactor is not particularly restricted provided that it is capable of varying the flow in response to external control signals. For example, variable fluid-delivery apparatus 5, 5′ and 5″ such as metering pumps driven by a pulse motor can be used. Further, the flow rates and the concentrations of solutions of each substrate can be varied by providing automatic control valves (not shown in the drawing) between vessels 2, 3 and 4 containing the solutions of each substrate and variable fluid-delivery apparatus 5, 5′ and 5″, and controlling opening and closing of the automatic control valves by external signals. The automatic control valves may be electromagnetic valves. Further, the reactor may be used, of course, at an ambient temperature. However, it is preferable to add a means for maintaining temperature. The automatic sampling apparatus 6 and the analyzing apparatus 7 for the reacting solution from the reactor are not particularly restricted, provided that AMP, ADP and ATP can be detected. An example of a preferred system is an high performance liquid chromatographic apparatus.

In the present invention, in order to carry out industrially, stably and economically substantial 100% conversion of AMP into ATP over a long period of time, it is preferred to control the concentration of ATP so as to satisfy the formula (a) above, and it is particularly preferred to control the concentration of ATP so as to be

$$0.08\times\frac{5+r^2}{r^2}\times AMP$$

or less in addition to the formula (a), the following method can be used. Namely, control may be carried out by a method which comprises previously feeding the formula (a) and data necessary for operation to the arithmetical control apparatus 8, carrying out operation of conversion of AMP into ATP from the above-described formula and data and analyzed data from the analyzing system, and sending signals from the arithmetical control apparatus 8 to at least one of the above-described variable fluid-delivery apparatus 5, 5′ and 5″ and the automatic control valves to vary the flow rate or the concentration so as to satisfy the formula (a). In this case, "data necessary for operation" means for concentrations of the AMP and ATP as raw materials and the ratio of the immobilized enzyme activity of the enzyme for converting ADP into ATP to the immobilized enzyme activity of the enzyme for converting AMP into ADP, and "analyzed data" means the concentrations of AMP, ADP and ATP in the reacting solution from the reactor. Further, the arithmetical control apparatus 8 refers to an apparatus having an arithmetical function and a function of sending control signals to an external apparatus. For example, a micro-computer can be used. Further, the "immobilized enzyme activity" means the activity of the immobilized enzyme,. For example, in case of adenylate kinase, activity in the direction of AMP+ATP→2ADP is intended. With respect to acetate kinase, activity in the direction of ADP+acetyl phosphate→ATP+acetic acid is intended. In order to measure the activity, a desired amount of the immobilized enzyme, for example, 5 to 10 µl according to degree of activity, is added to a solution for measuring activity, and activity is measured by pursuing it as a change in absorbance by means of a spectrophotometer in the same manner as in case of a free enzyme. 1 unit of enzyme activity means the amount of required to produce 1 micromole of ADP per minute at 30°C in case of adenylate kinase and the amount of required to produce 1 micromole of ATP per minute in case in acetate kinase.

The ATP used in the present invention may be the ATP which is the final conversion product of the above-described reaction which is utilized by means of circulation. In this case, it is sufficient to supply only acetyl phosphate as the phosphoric acid donor.

In the present invention, the enzymic reactions for synthesizing physiologically active substances using ATP as an energy source (hereinafter referred to as reaction system for physiologically active substance) may use one or more of the above-described synthesis reactions as the main reactions. Examples of these include a reaction for synthesizing peptide and peptide derivatives from amino acids by means of aminoacyl t-RNA synthetase, a reaction for synthesizing acetyl CoA or acyl CoA from acetic acid or aliphatic acid and CoA by means of acetyl CoA synthetase or acyl CoA synthetase, a reaction for synthesizing L-pantothenic acid from pantoic acid and β-alanine by means of pantothenic acid synthetase, a reaction for synthesizing guanylic acid from xanthylic acid and ammonia or glutamine by means of guanylic acid synthetase, a reaction for synthesizing asparagine from aspartic acid and ammonia by means of asparagine synthetase, a reaction for synthesizing acyl CoA from carboxylic acid and CoA by means of butyryl CoA synthetase, a reaction for synthesizing O-D-alanyl-poly(ribitol phosphate) from D-alanine and poly(ribitol phosphate) by means of D-alanyl-poly(ribitol phosphate) synthetase and a reaction for synthesizing $NAD^+$ from deamido $NAD^+$ and L-glutamine by means of $NAD^+$ synthetase, etc.

In the present invention, ATP is consumed in the above-described reaction system for making a physiologically active substance resulting in the formation of AMP. This resulting AMP is converted back into ATP using a combination of an enzyme which converts AMP into ADP and an enzyme which converts ADP into ATP, as described above (hereinafter referred to as the reaction system for reproduction of ATP).

In order to synthesize a physiologically active substance from AMP as a raw material in the above-described reaction system for physiologically active substance, a reactor is first prepared. The reactor may be a membrane type reactor or a column type reactor. The membrane type reactor is particularly effective to use when the physiologically active substance is a low molecular weight material. In this case, since the enzymes are high molecular weight materials, they are retaining in the reactor by the membrane. The resulting AMP is eluted from the reactor because it is a low molecular weight material. After it has been separated from the physiologically active substance by a simple operation such as ion-exchange chromatography, etc., it is sent back to the reactor, by which it is possible to reproduce ATP. Further, if the so-called water-soluble high molecular weight ATP which is obtained by previously introducing a suitable spacer into ATP and bonding to a water-soluble high molecular weight substance is used, the above-described operation for separation is not required. Various materials having a molecular weight of 1,000 to 500,000 may be used as the water-soluble high molecular weight substance. For example, it is possible to use polysaccharides such as a soluble dextran, vinyl polymer derivatives such as polyacrylamide derivatives or polyacrylic acid derivatives, and polyether derivatives such as polyethylene glycol derivatives, etc.

The column type reactor can be used without regard to the type of physiologically active substance. When a column reactor is used, each enzyme is packed in the column in a form of a so-called immobilized enzyme which is prepared by bonding to, including in or absorbing in a suitable carrier as described above. In this reactor, the resulting AMP flows out of the reactor whether it is a high molecular weight polymer or not, but it can be sent back to the reactor after being separated from the physiologically active substance in the same manner as described above. Further, in case of water-soluble high molecular weight ATP, the operation for separation can be easily carried out, because it can be separated by membrane separation.

The above-described reactor has been explained on the assumption that the operation is carried out continuously, and other reactors may be designed on the basis of such an idea. If necessary, a batch type reactor may be used in order to carry out a batchwise operation.

In the present invention, the reaction system for physiologically active substance and the reaction system for reproduction of ATP may be operated by combining them using different reactors, respectively. Further, the reaction system for physiologically active substance and the reaction system for reproduction of ATP may be operated in the same reactor. However, in order to synthesize the physiologically active substance efficiently, it is desirable to supply AMP produced in the reaction system for physiologically active substance to the reaction system for reproduction of ATP together with ATP, in both cases. In such cases, it is preferred that the ratio of AMP to ATP is in the range shown by the above-described formula (a).

In order to operate the reaction system for producing a physiologically active substance and the reaction system for reproduction of ATP by combining them using different reactors, it is possible to use, for example, the following method. First, to a reactor of the reaction system for reproduction of ATP, 0.1 $\mu$M to 4 M (preferably 1 $\mu$M to 2 M and, more preferably 10 $\mu$M to 500 mM) of AMP, 0.1 $\mu$M to 500 mM (preferably 1 $\mu$M to 400 mM and, more preferably 10 $\mu$M to 300 mM) of acetyl phosphate and ATP (in an amount of, preferably, 4% or more based on AMP as shown in the formula (a), though it varies according to the ratio in the reaction system for reproduction of ATP) are fed to one end of the reactor together with AMP to carry out conversion of AMP$\rightarrow$ADP$\rightarrow$ATP in the reactor. The reacting solution eluted from the reactor is analyzed by a suitable analyzing system, by which the concentrations of AMP, ADP and ATP and the conversion to ATP can be determined. In this case, the flow rate varies according to the size of the reactor, and, for example, a suitable flow rate can be selected from the linear velocity range of $1 \times 10^{-4}$ cm/hr to $1 \times 10^6$ m/hr. A part of the reacting solution (the concentration of ATP is desired to be 4% or more based on the concentration of AMP) is circulated back to the inlet of the reactor and feeding of the ATP solution fed in the initial stage is stopped. The larger part of the reacting solution is fed immediately to the reactor of the reaction system for the physiologically active substance whilst controlling the concentration thereof together with substrates for the reaction system for the physiologically active substance in the case where substances excepting ATP in the reacting solution, for example, acetic acid, do not inhibit the reaction system for the physiologically active substance. Alternatively, the larger part of the reacting solution is fed to an end of the reactor of the reaction system for the physiologically active substance together with a solution obtained by dissolving the substrates for the reaction system for the physiologically active substance after any inhibiting substances have been removed by a suitable separation means such as ion-exchange resin, etc. In this case, the flow rate varies according to the size of the reactor and a suitable flow rate may be selected, for example, from the linear velocity range of $1 \times 10^{-4}$ cm/hr to $1 \times 10^6$ m/hr. Concentrations of the substrates vary according to the physiologically active substance. When the solubility of the substrate is lower than the concentration of ATP fed from the reaction system for reproduction of ATP, the solubility of the substrate is the upper limit of concentration. Further, when the solubility of the substrate is higher than the concentration of ATP fed, the concentration similar to the concentration of ATP is the highest concentration of the substrate. Further, in the latter case, if the ATP fed is concentrated, the synthetic reaction can be carried out at a higher concentration. In this case, operation becomes discontinuous because of the operation for concentration. The reaction product (physiologically active substance) and AMP are separated from the eluted solution in the reactor by a suitable separation means such as ion-exchange resin, and AMP is sent back to an end of the reactor of the reaction system for reproduction of ATP, by which ATP is reproduced again.

When the reaction system for producing a physiologically active substance and the reaction system for reproduction of ATP are operated in the same reactor, the operation can be carried out in only when substrates in both enzymic systems do not inhibit the enzymic reaction systems of each other. Concentrations of substrates in both enzymic reaction systems are desired to be selected according to a relation between the concentration of ATP produced in the reaction system for reproduction of ATP and the solubility of the substrate in the reaction system for physiologically active substance. For example, when the solubility of the substrate in the reaction system for physiologically active substance is lower than the concentration of ATP, concentrations of AMP, ATP and acetyl phosphate may be selected such that the concentration of ATP produced in the reaction system for reproduction of ATP corresponds to the concentration of the substrate. In contrast with this, when the solubility of the substrate in the reaction system for physiologically active substance is higher than the concentration of ATP, it is desirable that the concentration of the substrate is allowed to correspond to the concentration of ATP produced in the reaction system for reproduction of ATP. It is preferred that the amount of ATP fed to the reaction system for reproduction of ATP together with AMP is more than 2 times (preferably more than 4 times and, more preferably, more than 5 times) that of the above-described case of using different reactors respectively. The flow rate varies according to the size of the reactor as well as the type of reactor, and a suitable flow rate can be selected from the linear velocity range, for example, $1\times10^{-4}$ cm/hr to $1\times10^{6}$ m/hr. Further, AMP and the physiologically active substance are separated from the reacting solution eluted from the reactor by means of a suitable separation means such as ion-exchange resin, etc., as described above, and AMP can be used again by returning it as a substrate to the inlet of the reactor.

In the present invention, the pH during the reaction varies according to enzyme used in the case of the reaction system for physiologically active substance, but a pH in the neutral range, namely, 6 to 11 (preferably 6.5 to 9.0) is generally used. In case of the reaction system for reproduction of ATP, a pH in the range of 6.5 to 11 (preferably 6.5 to 9.0 and, more preferably 7 to 8) is used. As a buffer solution, it is possible to use conventional solutions suitable for these pH ranges. For example, phosphates, imidazole salts, trishydrochloride, collidine salts and barbital hydrochloride, etc., can be used near pH 7. Further, the temperature for treatment can be selected in the room temperature to 50°C range. In case of the reaction system for reproduction of ATP, though the reaction for reproduction of ATP may be carried out at a higher temperature, it is preferred to operate at a temperature of 5°C or less, which is the maximum growth temperature of enzyme-producing microorganisms. Moreover, in order for the reaction of adenylate kinase and acetate kinase to proceed effectively in the reaction system for reproduction of ATP, various divalent metal ions can be used. As the divalent metal ions, magnesium and manganese ions are particularly recommended.

According to the present invention, changes in conversion shown in the prior conversion of AMP into ATP can be overcome, and AMP can be converted effectively, continuously and economically into ATP at a conversion of substantially 100% over a long period of time. In addition, ATP conversion can be kept stable for a long period of time because of having good operation properties. Further, it is an advantage of the present invention that ATP converted from AMP in the packed bed type reactor can be used as a phosphoric acid donor. Moreover, as a starting material, pure AMP is not required, and mixtures of AMP with ADP and ATP may be used if they are controlled so as to satisfy the formula (a). Accordingly, the process is very advantageous for industrial use. Furthermore, the reaction system for reproduction of ATP can be advantageously applied to the reproduction and utilization of ATP, and, from a different point of view, it can be thought of as a process for production of ATP using AMP as a raw material.

ATP is an important material as a medicine and has been produced industrially. However, the fermention process in the prior art has problems that by-products are easily formed and productivity is inferior. Consequently, the price of ATP has been high. However, according to the present invention, such problems can be eliminated and ATP having high purity can be supplied with good productivity.

According to the present invention, enzymic reaction using ATP as an auxiliary factor can be carried out continuously, effectively and economically by using the above-described reactors. Accordingly, it becomes possible to realize the operation of the so-called bioreactor wherein coupling reactions carried out in the living body are carried out as industrial chemical reactions outside the living body. Particularly, it is of great industrial value that protein synthesis reactions and peptide synthesis reactions by means of amino acid activating enzymes, which are the most important reactions in the living body, can be utilized for practical application.

In the following, the present invention is illustrated in greater detail in examples. (®indicates Reistered Trade Mark).

Examples 1—7

After 5 g of activated CH-Sepharose® 4B (produced by Pharmacia Fine Chemicals) was washed to swell, 2,000 units of acetate kinase obtained from *Bacillus stearothermophilus* NCA 1503 (optimum growth temperature: 60°C) (sold by Seikagaku Kogyo Co.) were added thereto and the reaction was carried out to obtain 1,000 units of immobilized acetate kinase. The same operation as described above was carried out using 250 units of adenylate kinase (sold by Seikagaku Kogyo Co.) instead of acetate kinase to obtain 100 units of immobilized adenylate kinase. The ratio of immobilized enzyme activity of acetate kinase to that of adenylate kinase in this case was 10.

A glass column having an inside diameter of 1.6 cm and a length of 10 cm was packed with both of these immobilized enzymes, and each substrate dissolved in a 25 mM imidazole hydrochloride buffer solution containing 10 mM magnesium chloride having a pH of 7.5 was fed to the column at a flow rate of 150 ml/hour. A variable fluid sending apparatus, an electromagnetic valve and a microcomputer were equipped on this column. The temperature in the column was kept at 30°C. Concentrations of AMP, ADP and ATP in the reacting solution eluted from the column were measured by a high performance liquid chromatographic apparatus. The concentration of AMP was fixed at 1.5 mM and the concentration of acetyl phosphate was fixed at 5 mM.

Conversion to ATP was then determined with varying the condition so as to satisfy the formula (a) by the microcomputer such that it was 0.063 mM ATP (ratio by concentration of ATP to AMP was 0.042: Example 1), 0.07 mM ATP (ratio by concentration of ATP to AMP was 0.047; Example 2), 0.13 mM ATP (ratio by concentration of ATP to AMP was 0.087; Example 3) and 0.19 mM ATP (ratio by concentration of ATP to AMP was 0.127; Example 4).

As a result, after feeding to the column no AMP was detected after only 20 minutes and 98.5% of ATP and 1.5% of ADP were detected.

Further, the same procedure was carried out with varying the concentration of ATP to 0.03 mM ATP (ratio of concentration of ATP to AMP was 0.02; Example 5), 0.024 mM ATP (ratio by concentration of ATP to AMP was 0.016; Example 6) and 0.014 mM ATP (ratio by concentration of ATP to AMP was 0.009; Example 7).

As a result, 95% of ATP, 3% of ADP and 2% of AMP were detected in Example 5, 89% of ATP, 8% of ADP and 3% of AMP were detected in Example 6, and 72% of ATP, 20% of ADP and 8% of AMP were detected in Example 7.

Example 8

After the reaction was initiated under the same condition as in Example 2, a solution eluted from the column after 20 minutes was circulated to feed to the column so that the ratio of ATP fed to the column to AMP was 0.047.

As a result, ATP was kept in the range of 98% to 98.5% over 5 hours after 20 minutes after the eluent from the reactor was used instead of ATP.

Example 9

A glass column having an inside diameter of 2.0 cm and a length of 12 cm was packed with 2,000 units of immobilized acetate kinase and 200 units of immobilized adenylate kinase obtained by the same method as in Example 1, and 3.0 mM of AMP, 0.13 mM of ATP (ratio by concentration of ATP to AMP was 0.043) and 10 mM of acetyl phosphate which were dissolved in a 50 mM imidazole-hydrochloride buffer solution containing 25 mM magnesium chloride and 0.04% sodium azide having a pH of 7.5 were fed to the column at a flow rate of 300 ml/hour, and flow rates and concentrations of AMP and ATP were maintained so as to satisfy the formula (a).

As a result, the conversion to ATP was kept in the range of 98.5% to 99.0% over 10 days after initiation of the reaction.

Example 10

After the reaction was initiated under the same condition as in Example 9, a solution eluted from the column after 30 minutes (containing 98% of ATP) was circulated to feed to the column so that the ratio by concentration of ATP fed to the column to AMP was 0.043.

As a result, the conversion to ATP was kept in the range of 98.2 to 98.7% over 10 days after initiation of the reaction.

Example 11

Adenylate kinase and acetate kinase materials derived from available *Bacillus stearothermophilus* (sold by Seikagaku Kogyo Co.) were obtained. Acetyl CoA synthetase, a material derived from available yeast (produced by Boehringer Mannheim Co.) was also obtained.

These three enzymes were immobilized on Sepharose® 4B as follows. Namely, after 5 g of activated CH-Sepharose® 4B (produced by Pharmacia Fine Chemicals) was washed to swell, 2,000 units of acetate kinase were added thereto and the reaction was carried out to obtain 1,000 units of immobilized acetate kinase. Likewise, 100 units of immobilized adenylase were obtained from 250 units of adenylate kinase, and 10 units of immobilized acetyl CoA synthetase were obtained from 100 units of acetyl CoA synthetase. A column for reproduction of ATP (inside diameter: 1.6 cm, length; 10 cm) was packed with the immobilized acetate kinase and the immobilized adenylate kinase, 6 mM of AMP, 0.3 mM of ATP and 25 mM of acetyl phosphate which were dissolved in a 25 mM imidazole hydrochloride buffer solution containing 10 mM magnesium chloride having a pH of 7.5 were fed to the column at a flow rate of 25 ml/hour. The reaction temperature in the column was kept at 30°C. The resulting ATP was sent back to the inlet of the column in an amount of 5% (0.3 mM) based on the concentration of AMP.

Further, another column (inside diameter: 1.0 cm, length: 9 cm) was packed with immobilized acetyl CoA synthetase. As a substrate, 4 mM of potassium acetate, 4 mM of reduction type CoA, lithium salt and 4 mM of $MgCl_2$ which were dissolved in a 100 mM imidazole hydrochloride buffer solution having a pH of 7.5 were flown at a flow rate of 25 ml/hour, which was mixed with an ATP solution eluted from the reaction system for production of ATP in a ratio of 1:1. The mixture was fed to a column packed with the immobilized acetyl CoA synthetase (flow rate: 50 ml/hour, reaction temperature: 37°C). Further, AMP was taken out from an eluate from the column by means of Dowex® 1-X8 (produced by The Dow Chemical Co.). After the pH and the concentration were controlled to desired values, it was fed to the column for reproduction of ATP by means of a pump. The amount of the resulting acetyl CoA was measured by a method which comprises sampling 0.05 ml of the elute from the column, adding 3 ml of 1 mM 5,5' - dithiobis(2 - nitrobenzoic acid) (DTNB) (pH 6.65, phosphoric acid buffer solution), determining the amount of un-reacting SH group by change in absorbance in 412 nm at room temperature after the passage of 20 minutes, and calculating therefrom.

As a result, 1.5 mM of acetyl CoA was formed after 30 minutes from the initiation of the reaction, and thereafter a stabilized state was maintained over 24 hours.

Example 12

A column (inside diameter: 1.8 cm, length: 12 cm) was packed with 2,000 units of immobilized acetate kinase, 200 units of immobilized adenylate kinase and 10 units of immobilized acetyl CoA synthetase which were obtained by the same methods as in Example 11, and 4 mM of AMP, 1.0 mM of ATP (25% based on the concentration of AMP), 25 mM of acetyl phosphate, 2.5 mM of potassium acetate and 2.5 mM of reduction type CoA lithium salt which were dissolved in a 100 mM imidazole hydrochloride buffer solution containing 10 mM magnesium chloride were fed thereto at a flow rate of 50 ml/hour. The reaction temperature in the column was kept at 37°C. AMP was separated from the solution eluted from the column by the same method as in Example 11 and it was sent back to the inlet for substrates. After 40 minutes from the initiation of the reaction, the formed acetyl CoA became 1.6 mM, and thereafter a stabilized state was kept over 15 hours.

Example 13

To the same column for reproduction of ATP as in Example 11, 6 mM of AMP, 0.05 mM of ATP and 25 mM of acetyl phosphate which were dissolved in a 25 mM imidazole hydrochloride buffer solution containing 10 mM of magnesium chloride having a pH of 7.5 were fed at a flow rate of 25 ml/hour. A part of the solution eluted from the column was sent back to the inlet of the column for reproduction of ATP by the same method as in Example 11 in an amount of 0.05 mM as a concentration of ATP. Then, synthesis of acetyl CoA was carried out by the same column packed with immobilized acetyl CoA synthesis enzyme as in Example 11.

As a result, 1.0 mM of acetyl CoA was formed after 30 minutes from the initiation of the reaction, and thereafter a stabilized state was maintained over 24 hours.

Example 14

Asparagine synthetase was produced from Lactobacillus arabinosus ATCC 8014 by carrying out ammonium sulfate fractionation, calcium phosphate gel treatment and gel filtration.

Using the same reaction system for reproduction of ATP as in Example 1, 4 mM of AMP, 0.3 mM of ATP (7.5% based on the concentration of AMP) and 16 mM of acetyl phosphate which were dissolved in a 25 mM imidazole hydrochloride buffer solution containing 10 mM manganese chloride having a pH of 7.5 were fed, and ATP was reproduced.

Further, 100 units of asparagine synthetase were dissolved in a 100 mM tris-hydrochloride buffer solution containing 4 mM of manganese chloride, and the resulting solution was enclosed in a membrane type reactor having an inside volume of 50 ml using an ultrafiltration membrane having a molecular weight of 30,000.

A 100 mM tris-hydrochloride buffer solution containing 4 mM manganese chloride in which 4 mM of ammonium chloride and 4 mM of L-aspartic acid were dissolved was mixed with an ATP solution obtained from the reaction system for reproduction of ATP in a ratio of 1:1 (by volume), and the resulting mixture was fed to the above-described reactor at a flow rate of 25 ml/hour. The reaction temperature in this case was kept at 37°C. AMP was separated from the elute by the same method as in Example 1, which was sent back to the reaction system for reproduction of ATP. The amount of the formed L-asparagine was determined by applying the elute to a high performance liquid chromatographic apparatus.

As conditions for the chromatographic apparatus in this case, Shimadzu Zorbax® ODS was used as the column and a mixture of 0.01 M sodium acetate (pH 4.5)/acetonitrile (55)45 by volume) was used as an elute at a flow rate of 1 ml/min, and detection was carried out by measuring an absorbance in 210 nm.

As a result, 1.5 mM of L-asparagine was formed after 30 minutes from the initiation of the reaction, and thereafter a stabilized state was maintained over 15 hours.

Example 15

Tyrosyl t-RNA synthetase was produced from *Bacillus stearothermophilus*; Deposition No. 5141 in Fermentation Research Institute by purifying through chromatography of DEAE®-cellulose (produced by Whatman Ltd.), hydroxyapatite (sold by Seikagaku Kogyo Co.) and DEAE-Sephadex (produced by Pharmacia Fine Chemicals, ammonium sulfate fractionation and chromatography of hydroxyapatite, DEAE®-Sephadex® and Sephadex® G-150 (produced by Parmacia Fine Chemicals).

Then, ATP (purity: 98%) was reproduced from AMP, a catalytic amount (5% based on the concentration of AMP) of ATP and acetyl phosphate by the same method as in Example 1, and it was used for the following reaction.

150 mg of the above-described purified tyrosyl t-RNA synthetase, 200 mg of magnesium chloride, 51 mg of ATP (purity: 98%), 0.5 mg of L-tyrosine, 100 units of pyrophosphatase (produced by Boehringer Mannheim) and 0.005 mg of dithiothreitol were dissolved in 70 ml of a 20 mM HEPES buffer solution (pH: 8.0), and they were allowed to react at 4°C for 15 minutes to obtain a reaction mixture. To the resulting reaction mixture 2 g of L-phenylalanine methyl ester was added and well blended. The mixture was allowed to stand for 1 day with maintaining the reaction temperature at 30°C to carry out the reaction.

To the resulting reacting solution, 100 ml of acetone was added. After precipitates were removed by centrifugal separation, the supernatant was concentrated to about 10 ml by an evaporator and processed by a high performance liquid chromatographic apparatus to separate a reaction product. As conditions for the chromatographic apparatus in this case, μ Bondapak® $C_{18}$ (produced by Waters Associates) were used as the column and development was carried out using a solvent mixture of 50 mM phosphoric acid buffer solution (pH 7.0)/acetonitrile (85/15) and detection was carried out by measuring an absorbance at 210 nm.

As a result, 0.22 mg of L-tyrosyl-L-phenylalanine methyl ester was obtained. Further, AMP eluted in the void section was separated at the same time by the same method as in Example 11 and sent back to the reaction system for reproduction of ATP to reproduce ATP.

Example 16

Methionyl t-RNA synthetase was obtained as a crude enzyme solution (purity: 10%) from available baker's yeast (produced by Oriental Yeast Co.) by operating with cellulose phosphate column chromatography.

Then ATP (purity: 98%) was reproduced from AMP, acatalytic amount (5% based on the concentration of AMP) of ATP and acetyl phosphate by the same method as in Example 1, and it was used for the following reaction.

1 g of the above-described crude methionyl t-RNA synthetase, 10 mg of magnesium chloride, 21 mg of ATP (purity: 98%), 0.5 mg of L-methionine, 5 units of pyrophosphatase (produced by Boehringer Mannheim) and 20 ml of mercaptoethanol were added to 15 ml of a 50 mM 2,5 - dimethylimidazole buffer solution having a pH of 9.0. After being allowed to react by the same method as in Example 15, the reaction mixture was treated with Sephadex® G-75 and elution was carried out with a HEPES buffer solution (pH:8.0). 30 ml of a fraction in the void section was collected and the reaction mixture was isolated. To the isolated mixture, 0.5 g of L-leucine ethyl ester was added in a solid state and the reaction was carried out at 25°C for 4 hours. To the resulting reaction product, 30 ml of acetone was added. After the formed precipitates were removed by centrifugal separation, the supernatant was concentrated to about 10 ml by an evaporator and separation was carried out by the same method as in Example 15 to obtain 0.92 mg of L-methionyl-L-leucine ethyl ester.

Further, ATP was reproduced from AMP by the same method as in Example 15.

**Claims**

1. A process for converting AMP into ATP comprising the steps of:

providing separately or together a first enzyme and a second enzyme in a reactor, the first enzyme being capable of converting AMP into ADP and having been produced from microorganisms having an optimum growth temperature of 50°C to 85°C, and the second enzyme being capable of converting ADP to ATP and having been produced from microorganisms having an optimum growth temperature of 50°C to 85°C;

adding AMP to the reactor to form ATP; and removing ATP from the reactor.

2. A process as claimed in Claim 1, further comprising the step of mixing ATP as a phosphoric acid donator with AMP and adding the resulting mixture to the reactor.

3. A process as claimed in Claim 2, wherein the mixing of AMP with ATP is carried out by controlling the concentration of ATP so as to satisfy the following formula (a):

$$0.15 \times \frac{5+r^2}{r^2} \times AMP \geqq ATP \geqq 0.04 \times \frac{5+r^2}{r^2} \times AMP$$

$$(a)$$

wherein AMP represents concentration of AMP (mM, ATP represents concentration of ATP (mM), and r represents a ratio of immobilized enzyme activity of the enzyme which converts ADP into ATP to immobilized enzyme activity of the enzyme which converts AMP into ADP, which is a positive number of 1 or more.

4. A process as clamed in Claim 1, wherein the first enzyme which converts AMP into ADP is adenylate kinase and the second enzyme which converts ADP into ATP is acetate kinase.

5. An apparatus for converting AMP into ATP comprising:

(a) an enzyme reactor having outlet conduit means and inlet conduit means, wherein said enzyme reactor contains:

(1) an enzyme which converts AMP into ADP, wherein said enzyme has been produced from microorganisms having an optimum growth temperature of 50°C to 85°C, and also contains

(2) an enzyme which converts ADP into ATP, wherein said enzyme has been produced from microorganisms having an optimum growth temperature of 50°C to 85°C;

(b) a source of AMP, wherein said source is connected to said enzyme reactor by said inlet conduit means;

(c) a source of phosphoric acid donor for converting AMP into ADP, wherein said source is connected to said enzyme reactor by said inlet conduit means;

(d) a source of phosphoric acid donor for converting ADP into ATP, wherein said source is connected to said enzyme reactor by said inlet conduit means;

(e) at least one variable fluid-delivery apparatus connected between each of said sources (b) (c) and (d), and said enzyme reactor, wherein said at least one variable fluid-delivery apparatus controls the flow of AMP from said source of AMP (b), the flow of phosphoric acid donor from said source of phosphoric acid donator (c) and the flow of phosphoric acid donor from said source of phosphoric acid donor (d), into said enzyme reactor;

(f) a recovery apparatus connected to said enzyme reactor outlet conduit means through;

(g) an automatic sampling apparatus;

(h) an analyzing apparatus which analyzes the concentrations of ATP, ADP and AMP in the reaction solution flowing from said enzyme reactor, wherein said analyzing apparatus provides signals indicative of said concentrations to;

(i) an arithmetical control apparatus which on the basis of signals received from said analyzing apparatus (h) and signals of previously prescribed values stored therein, provides signals to said at least one variable fluid sending apparatus (e) to control the amount of flow from said sources (b), (c) and (d) to said enzyme reactor so as to maintain the ATP conversion in said enzyme reactor or the ATP concentration in said enzyme reactor outlet conduit means at previously prescribed values.

6. An apparatus according to Claim 5, wherein the enzyme and the phosphoric acid donor for converting ADP into ATP are acetate kinase and acetyl phosphate, respectively, and the enzyme and the phosphoric acid donor for converting AMP into ADP are adenylate kinase and ATP, respectively,

7. An apparatus according to Claim 5, wherein the enzyme reactor is a column packed with enzymes immobilized on a water-insoluble carrier.

8. An apparatus according to Claim 5, wherein the analyzing apparatus is a high performance liquid chromatographic apparatus.

9. A process for producing a physiologically active substance by a multienzyme process which comprises (a) forming ATP from AMP using a combination of an enzyme which converts AMP into ADP and has been produced from microorganisms having an optimum growth temperature of 50°C to 85°C, and an enzyme which converts ADP into ATP and which has been produced from microorganisms having an optimum growth temperature of 50°C to 85°C, (b) synthesizing a physiologically active substance with the resulting ATP, converting the AMP resulting by the reaction in the step (b) into ATP by the reaction in the step (a), and repeatedly utilizing the converted ATP for synthesis of the physiologically active substance in the step (b).

10. A process for producing a physiologically active substance as claimed in Claim 9, wherein AMP formed by the reaction in the step (b) is applied to the reaction in the step (a) together with ATP to convert into ATP.

**Patentansprüche**

1. Verfahren zur Umwandlung von AMP in ATP, gekennzeichnet durch die Schritte:

Bereitstellen eines ersten Enzyms und eines zweiten Enzyms in einem Reaktor, und zwar entweder getrennt oder zusammen, wobei das erste Enzym in der Lage ist, AMP in ADP umzuwandeln und von Mikroorganismen mit einer optimalen Wachstumstemperatur von 50 bis 85°C produziert wurde, und das zweite Enzym in der Lage ist, ADP in ATP zumzuwandeln und von Mikroorganismen mit einer optimalen Wachstumstemperatur von 50 bis 85°C · produziert wurde;

Zugeben von AMP in den Reaktor unter Bildung von ATP; und

Enternen von ATP aus dem Reaktor.

2. Verfahren nach Anspruch 1, welches im weiteren den Schritt des Vermischens von ATP als einen Phosphorsäuredonor mit AMP und Zugabe des erhaltenen Gemisches in den Reaktor umfasst.

3. Verfahren nach Anspruch 2, wobei das Vermischen von AMP mit ATP ausgeführt wird unter Steuerung der ATP-Konzentration, so dass diese die folgende Gleichung (a) erfüllt:

$$0,15 \times \frac{5+r^2}{r^2} \times AMP \geqq ATP \geqq 0,04 \times \frac{5+r^2}{r^2} \times AMP \quad (a)$$

worin AMP die AMP-Konzentration (mM), ATP die ATP-Konzentration (mM) und r das Verhältnis von immobilisierter Enzymaktivität des Enzyms, welches ADP in ATP umwandelt, zu immobilisierter Enzymaktivität des Enzyms, welches AMP in ADP umwandelt, wobei dasselbe eine positive Zahl von 1 oder darüber bedeutet, darstellen.

4. Verfahren nach Anspruch 1, wobei das erste Enzym, welches AMP in ADP umwandelt, Adenylatkinase und das zweite Enzym, welches ADP in ATP umwandelt, Acetatkinase darstellt.

5. Vorrichtung zur Umwandlung von AMP in ATP, welche umfasst:

(a) einen Enzymreaktor mit Auslassleitungen und Einlassleitungen, wobei der genannte Enzymreaktor enthält:

(1) ein Enzym, welches AMP in ADP umwandelt, wobei das genannte Enzym von Mikroorganismen mit einer optimalen Wachstumstemperatur von 50 bis 85°C produziert wurde, und im weiteren enthält

(2) ein Enzym, welches ADP in ATP umwandelt, wobei das genannte Enzym von Mikroorganismen mit einer optimalen Wachstumstemperatur von 50 bis 85°C produziert wurde;

(b) eine AMP-Quelle, wobei die genannte Quelle mit dem genannten Enzymreaktor mittels Einlassleitungen verbunden ist;

(c) eine Quelle eines Phosphorsäuredonors zur Umwandlung von AMP in ADP, wobei die genannte Quelle mit dem Enzymreaktor mittels Einlassleitungen verbunden ist;

(d) eine Quelle eines Phosphorsäuredonors zur Umwandlung von ADP in ATP, wobei die genannte Quelle mit dem Enzymreaktor mittels Einlassleitungen verbunden ist;

(e) mindestens eine verstellbare Vorrichtung zur Flüssigkeitsabgabe, die zwischen jeder der gennannten Quellen (b), (c) und (d) und dem Enzymreaktor angebracht ist, wobei diese mindestens eine verstellbare Vorrichtung zur Flüssigkeitsabgabe den AMP-Fluss von der genannten AMP-Quelle (b), den Fluss des Phosphorsäuredonors von der genannten Quelle des Phosphorsäuredonors (c) und den Fluss des Phosphorsäuredonors von der genannten Quelle des Phosphorsäuredonors (d) in den genannten Enzymreaktor steuert;

(f) eine Gewinnungsvorrichtung, welche mit der genannten Enzymreaktor - Auslassleitung verbunden ist durch

(g) eine automatische Probeentnahme - Vorrichtung;

(h) eine Analysiervorrichtung, wobei dieselbe Signale liefert, die den Konzentrationen an zu analysierendem ATP, ADP und AMP in der Reaktionslösung entsprechen und die Reaktionslösung von dem genannten Enzymreaktor zu

(i) einer arithmetischen Kontrollvorrichtung fliesst, die basierend auf den von der Analysiervorrichtung (h) gelieferten Signalen und den vorher eingegebenen, in derselben gespeicherten Signalwerten Signale an mindestens einen der verstellbaren flüssigkeitsabgebenden Vorrichtungen (e) liefert, um die Fliessgeschwindigkeit von den genannten Quellen (b), (c) und (d) zu dem Enzymreaktor so zu steuern, dass die ATP-Umwandlung in dem genannten Enzymreaktor oder die ATP-Konzentration in der Enzymreaktor - Auslassleitung auf vorher eingegebene Werte gehalten wird.

6. Vorrichtung nach Anspruch 5, wobei das Enzym und der Phosphorsäuredonor zur Umwandlung von ADP in ATP jeweils Acetatkinase und Acetylphosphat darstellen und das Enzym und der Phosphorsäuredonor zur Umwandlung von AMP in ADP jeweils Adenylatkinase und ATP darstellen.

7. Vorrichtung nach Anspruch 5, wobei der Enzymreaktor eine Säule darstellt, die mit Enzymen, welche auf einem wasserunlöslichen Träger immobilisiert sind, gefüllt ist.

8. Vorrichtung nach Anspruch 5, wobei die Analysevorrichtung eine Hochleistungs - Flüssigchromatografie - Vorrichtung ist.

9. Verfahren zur Herstellung einer physiologisch aktiven Substanz mittels eines Multienzym - Verfahrens, gekennzeichnet durch

(a) Bildung von ATP aus AMP unter Verwendung einer Kombination eines Enzyms, welches AMP in ADP umwandelt und von einem Mikroorganismus mit einer optimalen Wachstumstemperatur von 50 bis 85°C hergestellt wurde, und einem Enzym, welches ADP in ATP umwandelt und von einem Mikroorganismus mit einer optimalen Waschstumstemperatur von 50 bis 85°C hergestellt wurde,

(b) Synthetisieren einer physiologisch aktiven Substanz mit dem erhaltenen ATP, Umwandlung des bei der Reaktion in Schritt (b) erhaltenen AMP im ATP durch Reaktion in Schritt (a) und wiederholte Verwendung des umgewandelten ATP zur Synthese der physiologisch aktiven Substanz in Schritt (b).

10. Verfahren zur Herstellung einer physiologisch aktiven Substanz nach Anspruch 9, wobei das bei der Reaktion in Schritt (b) gebildete AMP verwendet wird zur Reaktion in Schritt (a) zusammen mit ATP zur Umwundlung in ATP.

**Revendications**

1. Un procédé pour la conversion de l'AMP en ATP comprenant les stades consistant à:

placer séparément ou simultanément une première enzyme et une seconde enzyme dans un réacteur, la première enzyme étant capable de transformer l'AMP en ADP et ayant été produite à partir de micro-organismes ayant une température optimale de croissance de 50°C à 85°C et la seconde enzyme étant capable de transformer l'ADP en ATP et ayant été produite à partir de microorganismes ayant une température optimale de croissance de 50°C à 85°C;

ajouter de l'AMP au réacteur pour former de l'ATP; et évacuer l'ATP de réacteur.

2. Un procédé comme revendiqué dans la revendication 1 comprenant de plus le stade de mélange d'ATP comme donneur d'acide phosphorique avec de l'AMP et addition du mélange obtenu au réacteur.

3. Un procédé comme revendiqué dans la revendication 2, dans lequel le mélange de l'AMP avec l'ATP est effectué par ajustement de la concentration de l'ATP de façon à satisfaire à la formule (a) suivante:

$$0,15 \times \frac{5+r^2}{r^2} \times AMP \geqq ATP \geqq 0,04 \times \frac{5+r^2}{r^2} \times AMP$$

(a)

dans laquelle AMP représente la concentration de l'AMP (mM), ATP représente la concentration de l'ATP (mM) et r représente un rapport de l'activité d'enzyme immobilisée de l'enzyme qui transforme l'ADP en ATP à l'activité d'enzyme immobilisée de l'enzyme qui transforme l'AMP en ADP, qui est un nombre positif de 1 ou plus.

4. Un procédé comme revendiqué dans la revendication 1, dans lequel la première enzyme qui transforme l'AMP en ADP est l'adénylate-kinase et la seconde enzyme qui transforme l'ADP en ATP est l'acétate-kinase.

5. Un appareil pour la conversion de l'AMP en ATP comprenant:

(a) un réacteur enzymatique ayant une canalisation de sortie et une canalisation d'entrée, ledit réacteur enzymatique contenant:

(1) une enzyme qui transforme l'AMP en ADP, ladite enzyme ayant été produite à partir de microorganismes ayant une température optimale de croissance de 50°C à 85°C et contenant également

(2) une enzyme qui transforme l'ADP en ATP, ladite enzyme ayant été produite à partir de microorganismes ayant une température optimale de croissance de 50°C à 85°C;

(b) une source d'AMP, ladite source étant raccordée audit réacteur enzymatique par ladite canalisation d'entrée;

(c) une source de donneur d'acide phosphorique pour transformer l'AMP en ADP, ladite source étant raccordée audit réacteur enzymatique par ladite canalisation d'entrée;

(d) une source de donneur d'acide phosphorique pour transformer l'ADP en ATP, ladite source étant raccordée audit réacteur enzymatique par ladite canalisation d'entrée;

(e) au moins un appareil d'apport variable de fluide raccordé entre chacune desdites sources (b), (c) et (d) et ledit réacteur enzymatique, ce ou ces appareils ajustant le débit d'AMP à partir de ladite source d'AMP (b), le débit de donneur d'acide phosphorique à partir de ladite source de donneur d'acide phosphorique (c) et le débit de donneur d'acide phosphorique à partir de ladite source de donneur d'acide phosphorique (d) dans ledit réacteur enzymatique;

(f) un appareil de récupération raccordé à ladite canalisation de sortie du réacteur enzymatique par l'intermédiaire de

(g) un appareil d'échantillonnage automatique;

(h) un appareil d'analyse qui analyse la concentration de l'ATP, l'ADP dans la solution réactionnelle sortant dudit réacteur enzymatique, cet appareil d'analyse fournissant des signaux indiquant lesdites concentrations à:

(i) un appareil de régulation arithmétique qui selon les signaux reçus à partir dudit appareil d'analyse (h) et les signaux de valeurs précédemment prescrites qui y sont stockées fournit les signaux audit appareil unique ou multiple d'apport variable de fluide (e) pour ajuster l'importance de l'écoulement à partir desdites sources (b), (c) et (d) vers ledit réacteur enzymatique afin de maintenir à des valeurs préalablement prescrites la conversion en ATP dans ledit réacteur enzymatique ou la concentration de l'ATP dans ladite canalisation de sortie du réacteur enzymatique.

6. Un appareil selon la revendication 5 dans lequel l'enzyme et le donneur d'acide phosphorique pour la conversion de l'ADP en ATP sont respectivement l'acétate-kinase et l'acétyl-phosphate et l'enzyme et le donneur d'acide phosphorique pour la conversion de l'AMP en ADP sont respectivement l'adénylate-kinase et l'ATP.

7. Un appareil selon la revendication 5, dans lequel le réacteur enzymatique est une colonne garnie d'enzymes immobilisées sur un support insoluble dans l'eau.

8. Un appareil selon revendication 5, dans lequel l'appareil d'analyse est un appareil de chromatographie liquide haute performance.

9. Un procédé pour la production d'une substance physiologiquement active qui consiste à (a) former de l'ATP à partir d'AMP en utilisant une combinaison d'une enzyme qui transforme l'AMP en ADP et qui a été produite à partir de microorganismes ayant une température optimale de croissance de 50°C à 85°C et d'une enzyme qui transforme l'ADP en ATP et qui a été produite à partir de microorganismes ayant une température optimale de croissance de 50°C à 85°C, (b) synthétiser une substance physiologiquement active avec l'ATP obtenu, transformer l'AMP résultant de la réaction dans le stade (b) en ATP par réaction dans le stade (a) et utiliser de façon répétée l'ATP obtenu per conversion pour la synthèse de la substance physiologiquement active dans le stade (b).

10. Un procédé pour la production d'une substance physiologiquement active comme revendiqué dans la revendication 9 dans lequel l'AMP formé par la réaction dans le stade (b) est appliqué à la réaction dans le stade (a) avec de l'ATP pour la conversion en ATP.